# FASCICULE DE BREVET EUROPEEN

(11) **EP 3 410 976 B1**
(45) Date de publication et mention de la délivrance du brevet: **31.07.2019**
(21) Numéro de dépôt: 17707613.0
(22) Date de dépôt: 01.02.2017
(51) Int. Cl.: A61B 90/30, A61C 19/04, A61C 19/05, A61B 90/35

(54) **PROCEDE DE CONTROLE DE L'ALIGNEMENT, DE L'EQUILIBRAGE ET DE LA SYMETRIE D'UN VISAGE**
VERFAHREN ZUR KONTROLLE DER AUSRICHTUNG, BALANCE UND SYMMETRIE EINES GESICHTS
METHOD FOR CONTROLLING THE ALIGNMENT, BALANCE AND SYMMETRY OF A FACE

(30) Priorité: 01.02.2016 FR 1650792
(43) Date de publication de la demande: 12.12.2018
(73) Titulaire: One Ortho, 69230 Saint-Genis-Laval (FR)
(72) Inventeur: ALEPEE, Christophe, 69003 Lyon (FR); CASIMIRO, Romeo, 69230 Saint Genis Laval (FR)
(74) Mandataire: Cabinet Laurent & Charras
(86) Numéro de dépôt international: PCT/FR2017/050222
(87) Numéro de publication internationale: WO 2017/134385

(56) Documents cités:
- WO-A2-2013/067606
- US-A1- 2009 162 809

## Description

### DOMAINE TECHNIQUE

La présente invention se rapporte au secteur technique de la chirurgie maxillo-faciale ou orthognathique, c'est-à-dire la chirurgie des malformations congénitales ou acquises des mâchoires, et concerne plus particulièrement un procédé de contrôle de l'alignement, de l'équilibrage, et de la symétrie d'un visage d'un patient.

En effet, lorsque l'orthodontie n'est pas suffisante pour réaligner les dents, il faut parfois recourir à la chirurgie pour intervenir directement sur le positionnement des os de la mâchoire, à savoir l'os maxillaire et l'os mandibulaire.

Lors de cette intervention chirurgicale, il est nécessaire pour le chirurgien de contrôler l'alignement, l'équilibre et la symétrie du visage du patient.

### ART ANTERIEUR

Afin de visualiser l'alignement et l'équilibre du visage, et notamment dans le cas d'asymétrie faciale, il est connu que le chirurgien trace au feutre des lignes sur le visage du patient. Ces lignes lui permettent de modéliser les différents plans ou lignes souhaités en peropératoire, à savoir le plan sagittal médian, le plan d'occlusion et la ligne bi-pupillaire, et de les contrôler en postopératoire pour évaluer le réalignement de la mâchoire.

Pour rappel, le plan d'occlusion est un plan conventionnel sur lequel les surfaces occlusales maxillaires et mandibulaires d'un patient se rencontrent, et la ligne bi-pupillaire est la ligne définie entre les pupilles des yeux du patient.

Cependant, la technique de traçage au feutre ne permet pas un contrôle précis. En effet, un décalage de plus ou moins 2 mm peut être observé avec l'utilisation des lignes tracées sur le visage du patient. De plus ces lignes sont tracées à main levée, ce qui limite la précision.

Il est également connu que le chirurgien effectue un contrôle de l'alignement à l'oeil. Cependant, le contrôle à l'oeil est, quant à lui, dépendant de la position du chirurgien par rapport au patient, qui varie pendant l'opération. Ce contrôle n'est donc pas répétable dans le temps et d'un patient à un autre.

L'état de la technique divulgue quelques dispositifs, mis au point pour remédier aux problèmes de précision et de répétabilité du contrôle par le chirurgien.

Il est par exemple connu la demande de brevet américain publiée sous le numéro US2008187882 qui divulgue un dispositif de repérage et de mesure permettant de mesurer, de façon précise, les paramètres structurels du visage d'un patient. Le dispositif est constitué d'un cadre sur lequel sont fixés des moyens de matérialisation et de positionnement de différentes lignes et plan du visage, tels que la ligne d'incisives, la ligne bi-pupillaire, et le plan de Camper qui passe par le point sous-nasal et par le milieu du conduit auditif. Ce dispositif est particulièrement conçu pour faciliter les opérations de reconstruction et de réparation dentaires et parodontales, pour le rétablissement d'une harmonie esthétique du visage.

Cependant, ce dispositif ne permet au chirurgien de visualiser le plan d'occlusion, ni le plan sagittal médian.

Il est également connu la demande internationale de brevet publiée sous le numéro WO9600535. Cette demande divulgue un dispositif permettant de mesurer le plan d'occlusion grâce à un dispositif inséré dans la bouche du patient.

Ce dispositif permet de mesurer et donc de modéliser le plan d'occlusion mais ne permet pas de mesurer ou de matérialiser de façon précise le plan sagittal médian ou la ligne bi-pupillaire.

Il est aussi connu la demande internationale de brevet publiée sous le numéro WO2013067606 qui décrit une méthode pour ajuster les dents lors de chirurgie. Cette méthode met en oeuvre un dispositif permettant de mesurer différentes lignes et plans du visage, telles que la ligne bi-pupillaire, le plan sagittal médian et le plan de Camper. Cependant ce dispositif ne permet pas de mesurer ou matérialiser de façon précise le plan d'occlusion.

Le document US2009162809 A1 décrit un guide d'alignement d'empreinte dentaire comprenant un cadre qui maintient un porte-empreinte d'empreinte dentaire à une extrémité et un projecteur d'un faisceau lumineux en colonne à l'autre extrémité. Le faisceau, provenant généralement d'une diode laser, est divisé par une lentille prismatique en deux faisceaux orthogonaux en forme d'éventail qui projettent des lignes verticales et horizontales sur le visage du patient. Les lignes sont couplées de manière fixe à un porte-empreinte, indiquant ainsi la position du porte-empreinte dans la bouche du patient.

### EXPOSE DE L'INVENTION

L'un des buts de l'invention est donc de remédier au moins aux inconvénients précités en proposant un procédé permettant de matérialiser le plan sagittal médian, le plan d'occlusion, et la ligne bi-pupillaire sur le visage d'un patient afin de permettre à un chirurgien d'effectuer un contrôle de l'alignement, de l'équilibrage, et de la symétrie du visage.

Un autre objectif de l'invention est de fournir un tel procédé qui permet un contrôle précis, fiable, répétable et qui soit facile à mettre en oeuvre.

A cet effet il a été mis au point un procédé de contrôle de l'alignement, de l'équilibrage, et de la symétrie d'un visage d'un patient, remarquable en ce qu'il consiste à positionner, face à un patient, un dispositif comprenant une source lumineuse projetant, sur le visage du patient, une ligne de lumière verticale matérialisant l'intersection du visage avec un plan sagittal, et une ligne de lumière horizontale matérialisant l'intersection du visage avec un plan transversal. La source lumineuse est montée sur des moyens supports, avec capacité de translation dans des directions parallèles aux lignes de lumière projetées, et avec capacité de pivotement autour d'un axe parallèle à la ligne de lumière horizontale projetée, de sorte à visualiser, sur le visage, le plan sagittal médian en ajustant la position de la ligne de lumière verticale par une translation horizontale de la source lumineuse, et soit la ligne bi-pupillaire en ajustant la position de la ligne de lumière horizontale par une translation verticale de la source lumineuse, soit le plan d'occlusion en ajustant la position de la ligne de lumière horizontale par une translation verticale de la source lumineuse et, si nécessaire, par un pivotement de ladite source lumineuse autour de l'axe.

Le dispositif peut être posé ou fixé de toute manière appropriée à proximité dudit patient ou sur celui-ci.

La visualisation du plan sagittal médian, du plan d'occlusion, et de la ligne bi-pupillaire, aisément et directement sur le visage du patient, permet au chirurgien de contrôler facilement l'alignement, l'équilibrage, et la symétrie du visage du patient.

Selon une forme de réalisation particulière, et afin de permettre un contrôle précis, fiable et répétable, le dispositif est positionné face au patient en étant fixé sur un élément fixe à proximité du patient, sur un élément mobile, tel qu'un support de perfusion, à positionner à proximité du patient, ou sur le patient lui-même de sorte que la source lumineuse projette les lignes de lumière directement sur le visage du patient.

La source lumineuse peut être de tout type appropriée, l'essentiel réside dans le fait que ladite source soit capable d'émettre une lumière suffisamment puissance et nette pour projeter des lignes de lumières visibles sur le visage du patient. A titre d'exemple, la source lumineuse comprend au moins un émetteur L.A.S.E.R., ou au moins une diode électroluminescente.

La source lumineuse peut être alimentée par une prise branchée sur le réseau électrique. Avantageusement, et pour faciliter davantage sa mise en oeuvre et afin de rendre le dispositif autonome, la source lumineuse est alimentée par batteries ou par piles.

### DESCRIPTION SOMMAIRE DES FIGURES

D'autres caractéristiques et avantages de l'invention ressortiront clairement de la description qui en est réalisée ci-après, à titre indicatif et nullement limitatif, en référence aux figures annexées dans lesquelles :
- la figure 1 est une représentation schématique illustrant, en perspective, la source lumineuse du dispositif projetant les lignes de lumière de manière à matérialiser le plan sagittal médian et la ligne bi-pupillaire sur un visage d'un patient ;
- la figure 2 est une représentation schématique similaire à celle de la figure 1, illustrant la source lumineuse translatée horizontalement pour matérialiser le plan d'occlusion ;
- la figure 3 est une vue similaire à celle de la figure 2, illustrant la capacité de pivotement de la source lumineuse ;
- la figure 4 est une vue similaire à celle de la figure 3, illustrant le pivotement dans la source lumineuse dans un autre sens.

### DESCRIPTION DETAILLEE DE L'INVENTION

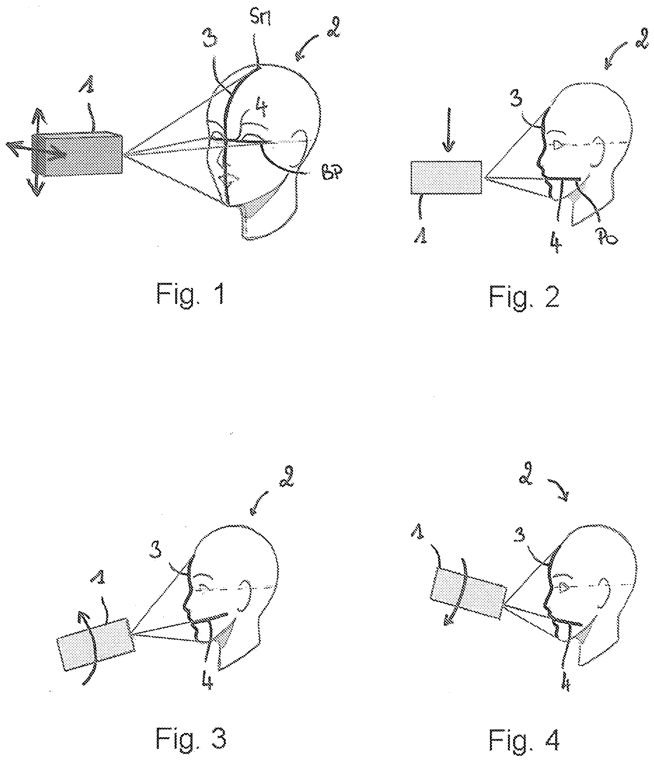

En référence aux figures 1 à 4, l'invention concerne un procédé permettant à un chirurgien, lors d'une opération de chirurgie maxillo-faciale ou orthognathique, de contrôler l'alignement, l'équilibrage et la symétrie du visage d'un patient (2) en préopératoire, peropératoire, et en postopératoire, en visualisant sur le visage du patient (2), le plan sagittal médian (SM), le plan d'occlusion (PO), et la ligne bi-pupillaire (BP).

A cet effet, le dispositif comprend une source lumineuse (1), branchée sur le réseau électrique ou alimentée par batteries ou par piles, apte à projeter, par l'intermédiaire d'au moins un laser ou au moins une diode électroluminescente, deux lignes de lumière orthogonales (3, 4) dans un plan frontal et notamment sur le visage d'un patient (2), à savoir une ligne verticale (3) et une ligne horizontale (4).

Plus précisément, la ligne de lumière verticale (3) permet de matérialiser l'intersection du plan frontal avec un plan sagittal, et la ligne de lumière horizontale (4) permet de matérialiser l'intersection du plan frontal avec un plan transversal.

La source lumineuse (1) est montée sur des moyens supports (non représentés), avec capacité de translation latérale parallèlement à la ligne de lumière horizontale (4) de sorte à ce que, par ajustement de la position de la source lumineuse (1) en translation latérale, la ligne de lumière verticale (3) projetée permet de visualiser le plan sagittal médian (SM) sur le visage du patient (2). De plus, la source lumineuse (1) est également montée avec capacité de translation verticale parallèlement à la ligne verticale (3) projetée, et avec capacité de pivotement autour d'un axe transversal parallèle à la ligne de lumière horizontale (4) projetée.

De cette manière, le chirurgien peut ajuster la position verticale de la source lumineuse (1) pour matérialiser la ligne bi-pupillaire (BP) au moyen de la ligne de lumière horizontale (4), ou pour matérialiser le plan d'occlusion (PO) en déplaçant verticalement la source lumineuse (1) et en la pivotant autour de son axe si nécessaire en fonction de la morphologie du patient (2).

Les moyens supports sont de tout type appropriés et sont notamment stérilisables et étanches. L'essentiel réside dans le fait qu'ils permettent de maintenir la source lumineuse (1) face à un patient (2) de manière à projeter les deux lignes de lumière (3, 4) sur son visage et de recevoir et supporter ladite source lumineuse (1) avec capacité de translation latérale et verticale et de pivotement pour matérialiser le plan sagittal médian (SM) et, à la convenance du chirurgien, la ligne bi-pupillaire (BP) ou le plan d'occlusion (PO).

Afin de permettre un contrôle précis, fiable et répétable, les moyens supports comprennent des moyens de fixation sur un élément fixe à proximité du patient, sur un élément mobile, tel qu'un support de perfusion, à positionner à proximité du patient, ou sur le patient (2) lui-même. Le dispositif selon l'invention peut en effet être fourni avec des moyens supports fixés sur un élément mobile, apte à être transporté dans un bloc opératoire. Les moyens supports sont fixés de sorte que la source lumineuse (1) projette les lignes de lumière (3, 4) directement sur le visage du patient (2). La forme de réalisation des moyens de fixation n'est pas limitée, l'essentiel réside dans le fait qu'ils doivent être conçus pour s'adapter et se fixer sur un quelconque dispositif déjà présent dans le bloc opératoire, ou destiné à être emmené dans le bloc opératoire, ou sur le patient (2) lui-même. Les moyens de fixation permettent une fixation stable par rapport au patient (2) afin d'assurer la répétabilité du contrôle de l'alignement du visage,

De ce qui précède, l'invention fournit un dispositif permettant de visualiser sur le visage d'un patient (2) d'une manière nette, précise et répétable, des plans/lignes de référence facilitant l'opération et le contrôle du chirurgien.

## Revendications

1. Procédé de contrôle de l'alignement, de l'équilibrage, et de la symétrie d'un visage d'un patient (2), ledit procédé consistant à positionner, face à un patient, un dispositif comprenant une source lumineuse (1) projetant, sur le visage du patient, une ligne de lumière verticale (3) matérialisant l'intersection du visage avec un plan sagittal, et une ligne de lumière horizontale (4) matérialisant l'intersection du visage avec un plan transversal, ladite source lumineuse (1) étant montée sur des moyens supports, avec capacité de translation dans des directions parallèles aux lignes de lumière (3, 4) projetées, et avec capacité de pivotement autour d'un axe parallèle à la ligne de lumière horizontale (4) projetée, de sorte à visualiser, sur le visage, le plan sagittal médian en ajustant la position de la ligne de lumière verticale par une translation horizontale de la source lumineuse, et soit la ligne bi-pupillaire en ajustant la position de la ligne de lumière horizontale par une translation verticale de la source lumineuse, soit le plan d'occlusion en ajustant la position de la ligne de lumière horizontale par une translation verticale de la source lumineuse et, si nécessaire, par un pivotement de ladite source lumineuse autour de l'axe.

2. Procédé selon la revendication 1, ***caractérisé* en ce que** le dispositif est positionné face au patient en étant fixé sur un élément positionné à proximité du patient (2).

3. Procédé selon la revendication 1, ***caractérisé* en ce que** le dispositif est positionné face au patient en étant fixé sur le patient (2) de sorte que la source lumineuse (1) projette les lignes de lumière (3, 4) directement sur le visage du patient (2).

## Patentansprüche

1. Verfahren zum Steuern der Ausrichtung, des Ausgleichs und der Symmetrie einer Gesichtsfläche eines Patienten (2), wobei das Verfahren darin besteht, einem Patienten gegenüber eine Vorrichtung zu positionieren, die eine Lichtquelle (1) umfasst, die auf die Gesichtsfläche des Patienten eine vertikale Lichtlinie (3) projiziert, die den Schnittpunkt der Gesichtsfläche mit einer Sagittalebene materialisiert, und eine horizontale Lichtlinie (4) projiziert, die den Schnittpunkt der Gesichtsfläche mit einer Transversalebene materialisiert, wobei die Lichtquelle (1) auf Trägermitteln montiert ist, mit Verschiebungsmöglichkeit in Richtungen parallel zu den projizierten Lichtlinien (3, 4) und mit Schwenkmöglichkeit um eine Achse parallel zu der projizierten horizontalen Lichtlinie (4), um auf der Gesichtsfläche die mittlere Sagittalebene zu visualisieren, indem die Position der vertikalen Lichtlinie durch eine horizontale Verschiebung der Lichtquelle angepasst wird, und um auf der Gesichtsfläche weiter entweder die Pupillenlinie durch Einstellen der Position der horizontalen Lichtlinie durch eine vertikale Verschiebung der Lichtquelle oder die Okklusionsebene durch Einstellen der Position der horizontalen Lichtlinie durch eine vertikale Verschiebung der Lichtquelle und, falls erforderlich, durch Schwenken der Lichtquelle um die Achse zu visualisieren.

2. Verfahren nach Anspruch 1, **dadurch gekennzeichnet, dass** die Vorrichtung dem Patienten gegenüber positioniert ist, wobei sie an einem Element befestigt wird, das in der Nähe des Patienten (2) positioniert ist.

3. Verfahren nach Anspruch 1, **dadurch gekennzeichnet, dass** die Vorrichtung dem Patienten gegenüber positioniert ist, wobei sie am Patienten (2) so befestigt ist, dass die Lichtquelle (1) die Lichtlinien (3, 4) direkt auf die Gesichtsfläche des Patienten (2) projiziert.

## Claims

1. Method of testing the alignment, balancing and symmetry of a patient's face (2), the said Method consisting in the positioning, in front of the patient, a device containing a light source (1) projecting a vertical line of light (3) onto the patient's face, materializing the intersection of the face with a sagittal plane, and a horizontal line of light (4) materializing the intersection of the face with a transversal plane, said source of light (1) being mounted on supporting means with a capability of translation in directions parallel to the projected lines of light (3, 4), and a capability of pivoting about an axis parallel to the projected horizontal line of light (4) to display, on the patient's face, the median sagittal plan while adjusting the position of the vertical line of light by a horizontal translation of the light source and even the bi-pupillary line by adjusting the position of the horizontal line of light by a vertical translation of the light source, or by the occlusion plane while adjusting the position of the horizontal line of light by the vertical translation of the light source and, if necessary, by pivoting the said source of light about the axis.

2. Method according to claim 1, ***characterized* in that** the device is positioned in front of the patient while being mounted on a device positioned near the patient (2).

3. Method according to claim 1, ***characterized* in that** the device is positioned in front of the patient while being attached to the patient (2) so that the light source (1) projects the lines of light (3, 4) directly onto the patient's face (2).
